# EUROPÄISCHE PATENTSCHRIFT

(11) **EP 1 518 874 B1**
(45) Veröffentlichungstag und Bekanntmachung des Hinweises auf die Patenterteilung: **13.12.2006**
(21) Anmeldenummer: 04018804.7
(22) Anmeldetag: 07.08.2004
(51) Int. Cl.: C08G 18/76, C07C 263/20

(54) **Monomerarmes Gemisch, enthaltend polymeres MDI**
Polymeric MDI having low monomer content
MDI polymere contenant un faible teneur en monomeres

(30) Priorität: 24.09.2003 DE 10344491; 29.10.2003 DE 10350816
(43) Veröffentlichungstag der Anmeldung: 30.03.2005
(73) Patentinhaber: BASF Aktiengesellschaft, 67056 Ludwigshafen (DE)
(72) Erfinder: Reese, Hans-Jürgen, 82140 Olching (DE); Murrar, Imbridt Dr., 01968 Senftenberg (DE); Wind, Michael Dr., 32689 Kalletal (DE)

(56) Entgegenhaltungen:
- WO-A1-02/070581
- GB-A- 1 263 439
- GB-A- 1 417 087
- US-A- 3 892 634

## Beschreibung

Die Erfindung betrifft ein monomerarmes Polymethylen-polyphenylenpolyisocyanatgemisch (als Polymer-MDI oder PMDI bezeichnet), enthaltend weniger als 0,4 Gew.-% 2-Kern-Methylendiphenyldiisocyanat (MDI), enthaltend 20 bis 45 Gew.-% 3-Kern MDI, 10 bis 30 Gew.-% 4-Kern MDI und 30 bis 60 Gew.-% 5-Kern und höherkerniges MDI, ein Verfahren zu dessen Herstellung und dessen Verwendung als Isocyanatkomponente für Einkomponenten- und Zweikomponentenschäume.

Einkomponentenschaumstoffe aus Aerosolbehältern sind im Bereich des Bauwesens häufig angewandte Montagemittel zum Einbau von Fenstern und Türen in Bauwerken sowie als Füllmaterial für bautechnisch bedingte Hohlräume oder Mauerdurchbrüche für Rohrinstallationen. Ein solcher Aerosolbehälter beinhaltet ein Prepolymer sowie Treibmittel und Zusätze. Durch Austragen seines Inhaltes mittels Treibmittel, seinem Aufschäumen durch Frothwirkung und durch seine Aushärtung mit Luftfeuchtigkeit, entsteht der gewünschte Schaumstoff.

Einkomponentenschaumstoffe auf der Basis von NCO-haltigen Prepolymeren sind die bekanntesten Schaumstoffe dieser Art. Es gibt hierbei unterschiedliche Produkte, die je nach Zusammensetzung zu harten bis weich elastischen Schäumen führen. Nachteilig bei all diesen Formulierungen ist, dass erhebliche Mengen an monomerem Isocyanat in diesen NCO-haltigen Prepolymeren vorhanden sind und damit während des Schäumprozesses ein gewisses Gefährdungspotential durch atembares Isocyanat besteht.

Bekannt sind in dieser Gruppe von Schäumen aber auch Formulierungen mit deutlich reduzierten Gehalten an freien monomeren Isocyanaten.
DE-A-4441696 beschreibt die Verwendung handelsüblicher Polymer-MDI's, wobei durch Destillation bei Temperaturen über 160°C und unter verminderten Druckbedingungen das monomere MDI teilweise entfernt und das übrigbleibende Sumpfprodukt als Isocyanatkomponente zur Herstellung eines 1 K-PUR-Montageschaumes verwendet wird.

Nachteilig an der in DE-A-4441696 beschriebenen Lösung ist, dass ein Polymer-MDI mit einem immer noch relativ hohen Restgehalt an monomerem Diisocyanat verwendet wird. Ein weiterer technischer Mangel ist die erhöhte Viskosität des eingesetzten Polymer-MDI's. Ferner wird dieses Isocyanat vor dessen Einsatz noch prepolymerisiert, was einen zusätzlichen Arbeitsschritt und einen weiteren negativen Viskositätsanstieg bedeutet. Der beschriebene Aerosolschaum auf Basis dieses relativ monomerarmen hochviskosen Polymer-MDI's weist deshalb unzureichende Eigenschaften auf.

Aufgabe der Erfindung war es deshalb, ein besonders geeignetes monomerarmes Polymethylen-polyphenylenpolyisocyanatgemisch (auch als Polymer-MDI-Gemisch oder PMDI-Gemisch bezeichnet) bereit zu stellen, d.h. ein Produkt mit niederer Viskosität bei gleichzeitig deutlich reduziertem technisch bedingtem Restmonomergehalt. Ferner war es Aufgabe der Erfindung, das PMDI-Gemisch in einem wesentlich verbesserten Verfahren von seinem Monomer-MDI-Anteil zu befreien.

Eine Aufgabe der Erfindung war es weiterhin, Isocyanatkomponenten zu erarbeiten, die technisch vorteilhaft handhabbar sind, insbesondere im Hinblick auf die Viskosität. Weiterhin war es die Aufgabe der Erfindung, 1-Komponenten bzw. 2-Komponentenschäume insbesondere in Form von Aerosolschäumen bei Verwendung oben genannter Isocyanatkomponenten zu entwickeln.

Die Aufgaben konnten gelöst werden, indem ausgehend von einem speziellen PMDI-Gemisch der 2-Kerngehalt mittels eines speziellen Verfahrens reduziert wurde.

Gegenstand der Erfindung ist somit ein Verfahren zur Herstellung eines monomerarmen Gemisches enthaltend Polymethylen-polyphenylenpolyisocyanate (PMDI), wobei
a) ein PMDI-Gemisch, umfassend
   35 ± 17 Gew.-% 2-Kern-Methylendiphenyldiisocyanat (MDI),
   26 ± 6 Gew.-% 3-Kern MDI,
   12 ± 4 Gew.-% 4-Kern MDI,
   5 ± 3 Gew.-% 5-Kern MDI,
   23 ± 10 Gew.-% 6-Kern und höherkerniges MDI,
   bereit gestellt wird und anschließend
b) das PMDI-Gemisch einem destillativen Trennverfahren unterworfen wird, wobei die Verdampfungstemperatur weniger als 160°C beträgt.

Im Rahmen dieser Erfindung wird unter Polymethylen-polyphenylenpolyisocyanat (PMDI) ein Gemisch, enthaltend Methylendiphenyldiisocyanat (als MDI oder auch als monomeres MDI bezeichnet) mit seinen höherkondensierten Homologen verstanden, d.h. PMDI ist ein Gemisch von Isocyanaten mit zwei und mehr aromatischen Kernen und NCO-Gruppen.

PMDI wird üblicherweise durch Phosgenierung eines Gemisches von Isomeren und Homologen des Diamino-diphenylmethans (MDA), welches durch Kondensation von Anilin und Formaldehyd erhältlich ist, hergestellt. Die Homologen bilden sich dadurch, dass die Kondensation nicht beim Zweikern-Produkt stehen bleibt, sondern in abnehmender Menge zu den drei- und höherkernigen Produkten weiterführt.

Das erfindungsgemäße Verfahren wird im Allgemeinen ausgehend von einem PMDI durchgeführt, das folgende Zusammensetzung hat:
ein PMDI-Gemisch, umfassend
35 ± 17 Gew.-% 2-Kern Methylendiphenyldiisocyanat (MDI),
26 ± 6 Gew.-% 3-Kern MDI,
12 ± 4 Gew.-% 4-Kern MDI,
5 ± 3 Gew.-% 5-Kern MDI,
23 ± 10 Gew.-% 6-Kern und höherkerniges MDI.

Die Gew.-%-Angaben beziehen sich jeweils auf 100 % PMDI-Gemisch.

In einer bevorzugten Ausführungsform wird das erfindungsgemäße Verfahren ausgehend von einem PMDI durchgeführt, das folgende Zusammensetzung hat:
43 ± 7 Gew.-% 2-Kern Methylendiphenyldiisocyanat (MDI),
24 ± 4 Gew.-% 3-Kern MDI,
10 ± 4 Gew.-% 4-Kern MDI,
5± 3 Gew.-% 5-Kern MDI,
20 ± 8 Gew.-% 6-Kern und höherkerniges MDI.

Besonders bevorzugt wird das erfindungsgemäße Verfahren ausgehend von folgender PMDI-Zusammensetzung durchgeführt:
47 ± 3 Gew.-% 2-Kern Methylendiphenyldiisocyanat (MDI),
23 ± 3 Gew.-% 3-Kern MDI,
10 ± 3 Gew.-% 4-Kern MDI,
5± 3 Gew.-% 5-Kern MDI,
15 ± 6 Gew.-% 6-Kern und höherkerniges MDI.

Im erfindungsgemäßen Verfahren wird das vorstehend genannte PMDI-Gemisch einem destillativen Trennverfahren unterworfen und man erhält das erfindungsgemäße monomerarme Polymethylen-polyphenylenpolyisocyanatgemisch (auch als monomerarmes PMDI-Gemisch bezeichnet).

Es ist ein wesentliches Merkmal der vorliegenden Erfindung, dass das destillative Trennverfahren bei Temperaturen unter 160°C durchgeführt wird. Bevorzugt wird das Trennverfahren bei Temperaturen von 100 bis 158°C, besonders bevorzugt von 120 bis 155°C, insbesondere von 130 bis 153°C durchgeführt.

Bei der Durchführung des erfindungsgemäßen Verfahrens beträgt der Druck in der Trennvorrichtung im Allgemeinen 0,001 mbar bis 10 mbar, bevorzugt 0,01 mbar bis 1 mbar, insbesondere 0,02 mbar bis 0,9 mbar.

Bei der Durchführung des erfindungsgemäßen Verfahrens beträgt des Weiteren die durchschnittliche Verweilzeit des zu trennenden Gemisches in der Trennvorrichtung im Allgemeinen 30 bis 1800 Sekunden, bevorzugt 50 bis 1600 Sekunden, besonders bevorzugt 60 bis 1500 Sekunden.

Bei der Durchführung des erfindungsgemäßen Verfahrens beträgt außerdem im Allgemeinen die Flächenbelastung des Verdampfers 0,05 bis 60 kg/m²h, bevorzugt 0,5 bis 50 kg/m²h. Die Temperatur im Kondensator beträgt im Allgemeinen 30 bis 60°C, bevorzugt etwa 45°C.

Gegenstand der Erfindung ist neben dem erfindungsgemäßen Verfahren auch das entsprechende Verfahrensprodukt, d.h. Gegenstand ist ein monomerarmes Polymethylen-polyphenylenpolyisocyanatgemisch, enthaltend weniger als 0,4 Gew.-% 2-Kern Methylendiphenyldiisocyanat (MDI), und enthaltend
20 bis 45 Gew.-% 3-Kern MDI,
10 bis 30 Gew.-% 4-Kern MDI und
30 bis 60 Gew.-% 5-Kern und höherkerniges MDI.

In einer bevorzugten Ausführungsform handelt es sich bei dem erfindungsgemäßen monomerarmen PMDI-Gemisch um ein Gemisch, enthaltend weniger als 0,1 Gew.-% 2-Kern Methylendiphenyldiisocyanat (MDI), und enthaltend
20 bis 40 Gew.-% 3-Kern MDI,
10 bis 30 Gew.-% 4-Kern MDI und
35 bis 60 Gew.-% 5-Kern und höherkerniges MDI.
Bei dem Gemisch dieser Ausführungsform handelt es sich um ein PMDI-Gemisch, bei welchem keine Gefahrenkennzeichnung aufgrund des 2-Kern MDI-Anteils notwendig ist, da dieser kleiner als 0,1 Gew.% ist.

Das erfindungsgemäße monomerarme PMDI-Gemisch weist im Allgemeinen eine Viskosität bei 25°C von 3 bis 150 Pas (Pascal-Sekunde) und bei 50°C von 100 bis 7000 mPas, bevorzugt eine Viskosität bei 25°C von 10 bis 100 Pas und bei 50°C von 300 bis 5000 mPas, besonders bevorzugt eine Viskosität bei 25°C von 12 bis 60 Pas und bei 50°C von 500 bis 4000 mPas auf, wobei die Viskosität nach DIN 53 018 bestimmt wurde.

Des Weiteren weist das erfindungsgemäße monomerarme PMDI-Gemisch im Allgemeinen eine Funktionalität von 3 bis 5, bevorzugt eine Funktionalität von 3,8 bis 4,4, besonders bevorzugt eine Funktionalität von 3,9 bis 4,3 auf.

Der NCO-Gehalt des erfindungsgemäßen PMDI-Gemisches beträgt im Allgemeinen 27 bis 33 Gew.-%, bevorzugt 28 bis 32,5 Gew.-%.

Gegenstand der Erfindung ist schließlich die Verwendung des erfindungsgemäßen monomerarmen PMDI-Gemisches zur Herstellung von Polyurethan-1-Komponentenschaum und/oder Polyurethan-2-Komponentenschaum.

Für die Verwendung des erfindungsgemäßen monomerarmen PMDI-Gemisches zur Herstellung einer Isocyanatkomponente für Polyurethan-1-Komponenten- und/oder Polyurethan-2-Komponentenschaum werden drei bevorzugte Ausführungsformen vorgeschlagen, wobei es sich um drei verschiedene Formulierungen einer Isocyanatkomponente handelt, welche das erfindungsgemäße monomerarme PMDI-Gemisch enthalten. Diese Isocyanatkomponenten zeichnen sich dadurch aus, dass sie je nach gewünschtem Typ und Eigenschaftsniveau des Endschaumstoffes ein spezielles Gemisch aus dem erfindungsgemäßen monomerarmen PMDI und anderen flüssigen inerten oder Isocyanatendgruppen tragenden Verbindungen darstellt. Wesentlich hierbei ist auch, dass es sich um Isocyanatkomponenten handelt, die bei Raumtemperatur, im Rahmen des Fertigungsprozesses der Aerosolschäume, bezüglich ihrer Viskosität technisch handhabbare Komponenten darstellen.

Die erste bevorzugte Ausführungsform betrifft eine Isocyanatkomponente zur Herstellung von 1-Komponenten-Polyurethanschaumstoffen, enthaltend
i) erfindungsgemäßes monomerarmes PMDI-Gemisch und
ii) Verdünnungsmittel.

Um eine Isocyanatkomponente in technisch handhabbarer Viskosität und ausreichendem Isocyanatgehalt zu erhalten, sollte der Gehalt an monomerarmem Polymer-MDI 50 bis 95 Gew.-%, bevorzugt 60 bis 90 Gew.-%, bezogen auf das Gesamtgewicht der Komponenten i) und ii) betragen.

Verdünnungsmittel sind im Allgemeinen Mittel, die als nichtreaktive Substanzen einen bedeutenden Vedünnungseffekt erzielen. Als geeignetes Verdünnungsmittel haben sich beispielsweise Trichlorpropylphosphat oder andere Alkyl-(oder Aryl)-phosphate erwiesen. Außerdem ist durch seine besonders verdünnende Wirkung Dimethoxymethan geeignet.

Bei Verwendung der Komponenten i) und ii) ist bevorzugt darauf zu achten, dass eine Isocyanatkomponente erhalten wird, welches in der Viskosität vorteilhaft handhabbar ist.

Bei Verwendung einer entsprechend abgestimmten Polyolkomponente sowie Treibgasen in der Aerosoldose wird daraus ein Isocyanatprepolymer erzeugt, welches trotz der erhöhten mittleren Funktionalität der monomerarmen im Vergleich zur herkömmlichen PMDI-Komponente zu einem hartelastischen Schaumstoff mit sehr guten Montageeigenschaften führt.

Als Polyole bzw. Polyolkomponente kommen (hier und bei den beiden nachstehend genannten Ausführungsformen) die üblicherweise in der Polyurethanchemie verwendeten Polyetherole, Polyesterole, Polymerpolyole oder Gemische davon zum Einsatz. Im allgemeinen werden die Polyole in einer Menge verwendet, dass das Verhältnis von gegenüber Isocyanaten reaktiven Gruppen (OH-Gruppen) zu Isocyanatgruppen 1 zu 1,1 bis 1 zu 10 beträgt.

Die zweite bevorzugte Ausführungsform betrifft eine Isocyanatkomponente zur Herstellung von 1-Komponenten-Polyurethanschaumstoffen, enthaltend
i) erfindungsgemäßes monomerarmes PMDI-Gemisch,
ii) Isocyanatprepolymere, erhältlich durch Umsetzung eines Polyisocyanats mit Polyolen und
iii) gegebenenfalls Verdünnungsmittel.

Im allgemeinen enthält 100 Gew.-% der Komponenten i) bis iii) folgende Zusammensetzung:
5 bis 95 Gew.-%, bevorzugt 10 bis 30 Gew.-% an monomerarmem PMDI (i),
0,1 bis 95 Gew.-%, bevorzugt 30 bis 70 Gew.-% Polyisocyanatprepolymere (ii),
0 bis 50 Gew.-%, bevorzugt 10 bis 40 Gew.-% an Verdünnungsmittel (iii).

Die Isocyanatprepolymere ii) werden nach allgemein aus dem Stand der Technik bekannten Verfahren hergestellt, beispielsweise indem das Polyisocyanat, insbesondere Diisocyanat, bei Temperaturen von etwa 50 bis 80°C mit Polyolen, insbesondere mit Diolen, zu einem Prepolymer umgesetzt wird. Um Nebenreaktionen durch Luftsauerstoff auszuschließen, sollte das Reaktionsgefäß mit einem Inertgas, vorzugsweise Stickstoff, gespült werden. Das Polyol-Polyisocyanat-Verhältnis wird so gewählt, dass der NCO-Gehalt des Prepolymeren 8 bis 30 Gew.-%, vorzugsweise 10 bis 25 Gew.-%, beträgt.

In einer bevorzugten Ausführungsform wird zur Gewährleistung der Monomerfreiheit für bestimmte Anwendungen das Prepolymere noch vor seiner Mischung mit dem erfindungsgemäßen monomerarmen PMDI und dem optionalen Verdünnungsmittel entmonomerisiert. Die Entmonomerisierung erfolgt bevorzugt durch Destillation. Das resultierende Prepolymer weist bevorzugt einen Gehalt an 2-Kern MDI von weniger als 0,1 Gew.-% und einen NCO-Gehalt von 1,5 bis 14 Gew.% auf.

Bei Verwendung der Komponenten i) bis iii) ist bevorzugt darauf zu achten, dass eine Isocyanatkomponente erhalten wird, welche in der Viskosität vorteilhaft handhabbar ist. Bei Verwendung einer entsprechend abgestimmten, vorstehend beschriebenen Polyolkomponente sowie Treibgasen in der Aerosoldose wird daraus ein Isocyanatprepolymer erzeugt, welches trotz der erhöhten mittleren Funktionalität der PMDI-Komponente zu einem Schaumstoff führt, der bei erhöhten Dehneigenschaften eine ausreichende Härte für Montagezwecke aufweist.

Die dritte bevorzugte Ausführungsform betrifft eine Isocyanatkomponente zur Herstellung von 1-Komponenten-Polyurethanschaumstoffen, enthaltend
i) erfindungsgemäßes monomerarmes PMDI-Gemisch,
ii) Isocyanatsilanprepolymere, erhältlich durch Umsetzung eines Polyisocyanats mit Polyolen und anschließender Umsetzung mit Silanen, die mindestens eine gegenüber Isocyanaten reaktive Gruppe aufweisen und
iii) gegebenenfalls Verdünnungsmittel.

Im allgemeinen enthält 100 Gew.-% der Komponenten i) bis iii) folgende Zusammensetzung:
5 bis 95 Gew.-%, bevorzugt 10 bis 30 Gew.-% an monomerarmem PMDI (i),
0,1 bis 95 Gew.-%, bevorzugt 30 bis 70 Gew.-% Silanendgruppen aufweisendes Prepolymer (ii) und
0 bis 50 Gew.-%, bevorzugt 10 bis 40 Gew.-% an Verdünnungsmittel (iii).

Unter Isocyanatsilanprepolymere werden Umsetzungsprodukte von vorstehend beschriebenen NCO-Prepolymeren mit Silanen, die mindestens eine gegenüber Isocyanaten reaktive Gruppe aufweisen, bevorzugt Aminosilane, insbesondere Amino(di- oder tri-methoxy- oder ethoxy)silane, verstanden. Sie werden im Allgemeinen in der Weise gefertigt, dass in der ersten Stufe ein NCO-Prepolymer aus Diisocyanaten und Polyolen, insbesondere Diolen, hergestellt wird. In einer zweiten Stufe wird das Prepolymer mit dem Silan, welches mindestens eine gegenüber isocyanaten reaktive Gruppe aufweist, bevorzugt Aminosilan, umgesetzt. In dieser kettenabbrechenden Reaktion werden anteilig die Isocyanatgruppen durch die Silangruppen ersetzt. Dabei kann der Umsatz sowohl äquivalent aber auch im Aminosilanunterschuss (es sind freie NCO-Gruppen vorhanden) erfolgen.

Zur gezielten Beeinflussung des Isocyanatgehaltes und der Funktionalität wird im Anschluss an den oben genannten Modifizierungsprozess das erfindungsgemäße monomerarme PMDI diesem Prepolymer zugesetzt.

Bei Verwendung der Komponenten i) bis iii) ist bevorzugt darauf zu achten, dass eine Isocyanatkomponente erhalten wird, welche in der Viskosität vorteilhaft handhabbar ist. Bei Verwendung einer entsprechend abgestimmten Polyolkomponente sowie Treibgasen in der Aerosoldose wird daraus ein Isocyanatprepolymer erzeugt, welches trotz der erhöhten mittleren Funktionalität der PMDI-Komponente zu einem Schaumstoff führt, der sowohl Isocyanat- als auch Silanendgruppen aufweist, womit sich die Möglichkeit erschließt, einen sowohl über Silangruppen als auch über Harnstoffgruppen aushärtenden Schaum mit guten mechanischen Eigenschaften zu erhalten. 3

Für alle drei Ausführungsformen gilt, dass der Prozess der Herstellung des 1-Komponenten- und/oder 2-Komponenten-Aerosolschaumes aus den oben genannten Komponenten die Reaktion der Komponenten bei stöchiometrischen Überschuss der Isocyanatkomponente in einem Druckbehälter in Gegenwart von Flüssiggasen umfasst. Das dabei entstehende in den Flüssiggasen gelöste Prepolymer wird im Falle der Verarbeitung zum Schaumstoff aus dem Druckbehälter entnommen, schäumt durch gelöstes Gas bei geänderten Druckbedingungen auf und härtet nachfolgend durch Luftfeuchtigkeit und/oder weiterer zugesetzter Feuchtigkeit zum fertigen Schaumstoff aus.

Die vorliegende Erfindung soll durch nachstehende Beispiele veranschaulicht werden.

Die nachfolgenden Beispiele gliedern sich in :

### Herstellung des monomerarmen Polymer-MDI

Herstellung einer Isocyanatkomponente 1 (Polymer-MDI & Verdünnungsmittel) und den auf dieser Komponente aufgebauten 1-Komponenten-Aerosolschaum

Herstellung einer Isocyanatkomponente 2 (Polymer-MDI & NCO-Prepolymer & Verdünnungsmittel) und den auf dieser Komponente aufgebauten 1-Komponenten-Aerosolschaum

Herstellung einer Isocyanatkomponente 3 (Polymer-MDI. & Silan-Prepolymer & Verdünnungsmittel) und den auf dieser Komponente aufgebauten 1 -Komponenten-Aerosolschaum

### Herstellung des monomerarmen Polymer-MDI

### Beispiel 1a

In einem Laborverdampfer vom Typ VKL 70-4 (VTA GmbH Deggendorf) wurden unter Einsatz von polymerem MDI (BASF: Lupranat M 10 R) das beanspruchte niederviskose monomerarme Polymer-MDI hergestellt. Das Ausgangsprodukt hatte die folgende Kernverteilung:
47 Gew.-% 2-Kern MDI
23 Gew.-% 3-Kern MDI
10 Gew.-% 4-Kern MDI
5 Gew.-% 5-Kern MDI
15 Gew.-% 6-Kern und höherkerniges MDI

Bei einer Verdampfungstemperatur von 153°C, einer Flächenbelastung des Verdampfers maximal 4,4 kg/m²·h, 0,04 mbar, einer Kondensatortemperatur von 45°C und einer Verweilzeit von 161 s wurde ein niederviskoses monomerarmes Polymer-MDI mit einem NCO-Gehalt von 29,2 Gew.-%, einer Viskosität von 2228 mPa·s bei 50°C, einem 2-Kern MDI-Anteil von 0,19 GPC-FI.-% und einer mittleren Funktionalität von 4,11 erhalten.

### Beispiel 1b

Das nach Beispiel 1 a eingesetzte polymere MDI wurde in einer alternativen Ausführungsform bei einer Verdampfertemperatur von 153°C, einem Druck von 0,06 mbar, einer mittleren Verweilzeit von 120 s und einer maximalen Flächenbelastung von 5,8 kg/m²h zu einem niedrigviskosen monomerarmen Polymer-MDI mit 29,5 Gew.-% freien NCO-Gruppen, 1610 mPa·s bei 50°C und 0,08 % 2-Kern MDI entmonomerisiert.

Herstellung der Isocyanatkomponente 1 :

### Beispiel 2

Aus 820 g des monomerarmen Polymer-MDI nach Beispiel 1 a und 180 g Trichlorpropylphosphat wird eine Isocyanatkomponente hergestellt. Diese Isocyanatkomponente weist bei einem Isocyanatgehalt von 23,9 Gew.-% eine Viskostät bei 25°C von 6600 mPas auf.

Herstellung der Polyolkomponente 1

### Beispiel 3

Aus 200 g eines Polyetherpolyols auf Basis Glyzerin/Propylenoxyd mit einem Hydroxylgehalt von 150 mgKOH/g, 550 g eines Polyetherpolyols auf Basis Glyzerin/Propylenoxyd/Ethylenoxyd mit einem Hydroxylgehalt von 42 mgKOH/g, 80 g eines bromierten Polyetherpolyols mit einem Hydroxylgehalt von 330 mgKOH/g, 80 g eines Polyethylenglykols mit einem Hydroxylgehalt von 187 mgKOH/g, 25 g eines Siliconschaumstabilisators sowie 8 g Dimorpholinodiethylether und 0,5 g eines Siliconentschäumers wird eine Polyolkomponente hergestellt.

Herstellung des 1-Komponenten-Aerosolschaumes 1 (in der Aerosoldose)

### Beispiel 4

170 g der Polyolkomponente nach Beispiel 3 werden in eine 1-Liter-Aerosoldose gefüllt. Dazu werden 480 g der Isocyanatkomponente nach Beispiel 2 dosiert und nachfolgend die Aerosoldose mit einem Kippventil verschlossen. Durch das Ventil werden nacheinander 152 g Tetrafluorethan und 65 g Dimethylether als Flüssiggase dosiert. Nach der Dosierung der Einzelkomponenten wird der Inhalt durch Schütteln homogenisiert. Die einsetzende Prepolymerreaktion ist erst nach 3 bis 4 Tagen abgeschlossen. Zum Zwecke der schnellen Testung wird die fertige Aerosoldose ca. 24 h bei 50°C im Wärmeschrank gelagert und ist nach dieser Zeit und der Abkühlung auf Raumtemperatur testbar.

Herstellung des 1-Komponenten-Aerosolschaumstoffes 1 (Testung des fertigen Schaumes)

### Beispiel 5

Papierflies wird auf einer festen Unterlage aufgebracht und angefeuchtet.
Auf das Kippventil der fertigen Aerosoldose nach Beispiel 4 wid das zugehörige Kunststoffröhrchen aufgeschraubt und der Inhalt der Aerosoldose, mit dem Ventil nach unten, durch Betätigung des Ventils über das Kunststoffröhrchen unter Aufschäumen entnommen.
Das aufschäumende Gemisch härtet zu einem dimensionsstabilen hartelastischen Schaumstoff. Durch ein weiteres Verschäumen des Restinhaltes in den Spalt einer üblichen Baukonstruktion aus Türrahmen und Mauerwerk konnte die Montageeignung nachgewiesen werden.

Herstellung einer Isocyanatkomponente 2

### Beispiel 6

Aus 155 g des monomerarmen Polymer-MDI nach Beispiel 1 a, 618 g eines monomerarmen Prepolymers auf Basis Methylendiphenyldiisocyanat (2,4'-MDI) und einem Polypropylenglykol mit einem Molekulargewicht von Mw = 450 g/mol, 167 g Trichlorpropylphosphat und 60 g Dimethoxymethan wird eine Isocyanatkomponente hergestellt. Diese Isocyanatkomponente weist bei einem Isocyanatgehalt von 9,70 Gew.-% NCO eine Viskostät bei 25°C von 9500 mPas auf.

Herstellung einer Polyolkomponente 2

### Beispiel 7

Aus 700 g eines Polyetherpolyols auf Basis Saccharose/Propylenoxyd mit einem Hydroxylgehalt von 410 mgKOH/g, 100 g eines Polyethylenglykols mit einem Hydroxylgehalt von 187 mgKOH/g, 100 g eines Siliconschaumstabilisators sowie 70 g Dimorpholinodiethylether und 1,2 g eines Siliconentschäumers wird eine Polyolkomponente hergestellt.

Herstellung des 1-Komponenten-Aerosolschaumes 2 (in der Aerosoldose)

### Beispiel 8

41 g der Polyolkomponente nach Beispiel 7 werden in eine 1-Liter-Aerosoldose gefüllt. Dazu werden 656 g der Isocyanatkomponente nach Beispiel 6 dosiert und nachfolgend die Aerosoldose mit einem Kippventil verschlossen. Durch das Ventil werden nacheinander 122 g Tetrafluorethan und 52 g Dimethylether als Flüssiggase dosiert. Nach der Dosierung der Einzelkomponenten wird der Inhalt durch Schütteln homogenisiert. Die einsetzende Prepolymerreaktion ist erst nach 3 bis 4 Tagen abgeschlossen. Zum Zwecke der schnellen Testung wird die fertige Aerosoldose ca. 24 h bei 50°C im Wärmeschrank gelagert und ist nach dieser Zeit und der Abkühlung auf Raumtemperatur testbar.

Herstellung des 1-Komponenten-Aerosolschaumstoffes 2 (Testung des fertigen Schaumstoffes)

### Beispiel 9

Papierflies wird auf einer festen Unterlage aufgebracht und angefeuchtet.
Auf das Kippventil der fertigen Aerosoldose nach Beispiel 8 wird das zugehörige Kunststoffröhrchen aufgeschraubt und der Inhalt der Aerosoldose, mit dem Ventil nach unten, durch Betätigung des Ventils über das Kunststoffröhrchen unter Aufschäumen entnommen.
Das aufschäumende Gemisch härtet zu einem dimensionsstabilen elastischen Schaumstoff.
Durch ein weiteres Verschäumen des Restinhaltes in den Spalt einer üblichen Baukonstruktion aus Türrahmen und Mauerwerk konnte die Montageeignung nachgewiesen werden, da dieser elastische Schaumstoff dennoch eine ausreichende Härte aufweist.

## Patentansprüche

1. Verfahren zur Herstellung eines monomerarmen Gemisches enthaltend Polymethylen-polyphenylenpolyisocyanate (PMDI), wobei
a) ein PMDI-Gemisch, umfassend
35 ± 17 Gew.-% 2-Kern-Methylendiphenyldiisocyanat (MDI),
26 ± 6 Gew.-% 3-Kern MDI,
12 ± 4 Gew.-% 4-Kern MDI,
5 ± 3 Gew.-% 5-Kern MDI,
23 ± 10 Gew.-% 6-Kern und höherkerniges MDI,
bereitgestellt wird und anschließend
b) das PMDI-Gemisch einem destillativen Trennverfahren unterworfen wird, wobei die Verdampfungstemperatur weniger als 160°C beträgt.

2. Verfahren nach Anspruch 1, **dadurch gekennzeichnet, dass** der Druck in der Trennvorrichtung 0,001 mbar bis 10 mbar, bevorzugt 0,01 mbar bis 1 mbar, beträgt.

3. Verfahren nach Anspruch 1, **dadurch gekennzeichnet, dass** die durchschnittliche Verweilzeit des zu trennenden Gemisches in der Trennvorrichtung 30 bis 1800 Sekunden, bevorzugt 50 bis 1600 Sekunden, beträgt.

4. Monomerarmes Polymethylen-polyphenylenpolyisocyanatgemisch, enthaltend weniger als 0,4 Gew.-% 2-Kern-Methylendiphenyldüsocyanat (MDI), enthaltend
20 bis 45 Gew.-% 3-Kem MDI,
10 bis 30 Gew.-% 4-Kern MDI und
30 bis 60 Gew.-% 5-Kern und höherkerniges MDI.

5. Monomerarmes PMDI-Gemisch nach Anspruch 4, **dadurch gekennzeichnet, dass** es eine Viskosität bei 25°C von 3 bis 150 Pa s und bei 50°C von 100 bis 7000 mPa s aufweist.

6. Monomerarmes PMDI-Gemisch nach Anspruch 4 oder 5, **dadurch gekennzeichnet, dass** es eine Funktionalität von 3,8 bis 4,4 aufweist.

7. Verwendung eines monomerarmen PMDI-Gemisches nach einem der Ansprüche 4 bis 6 zur Herstellung von Polyurethan-1-Komponentenschaum und/oder Polyurethan-2-Komponentenschaum.

8. Isocyanatkomponente zur Herstellung von Polyurethan-1-Komponenten- und/oder Polyurethan-2-Komponentenschaumstoffen, enthaltend
i) monomerarmes PMDI-Gemisch gemäß einem der Ansprüche 4 bis 6 und
ii) Verdünnungsmittel.

9. Isocyanatkomponente zur Herstellung von Polyurethan-1-Komponenten- und/oder Polyurethan-2-Komponentenschaumstoffen, enthaltend
i) monomerarmes PMDI-Gemisch gemäß einem der Ansprüche 4 bis 6,
ii) Isocyanatprepolymere, erhältlich durch Umsetzung eines Polyisocyanats mit Polyolen und
iii) gegebenenfalls Verdünnungsmittel.

10. Isocyanatkomponente zur Herstellung von Polyurethan-1-Komponenten- und/oder Polyurethan-2-Komponentenschaumstoffen, enthaltend
i) monomerarmes PMDI-Gemisch gemäß einem der Ansprüche 4 bis 6,
ii) Polyisocyanatsilanprepolymere, erhältlich durch Umsetzung eines Polyisocyanats mit Polyolen und anschließender Umsetzung mit Silanen
iii) gegebenenfalls Verdünnungsmittel.

## Claims

1. A process for preparing a low-monomer mixture comprising polymethylenepolyphenylene polyisocyanates (PMDI), which comprises
a) providing a PMDI mixture comprising
35 ± 17% by weight of 2-ring methylenediphenyl diisocyanate (MDI),
26 ± 6% by weight of 3-ring MDI,
12 ± 4% by weight of 4-ring MDI,
5 ± 3% by weight of 5-ring MDI,
23 ± 10% by weight of 6-ring and higher MDI
and subsequently
b) subjecting the PMDI mixture to a distillative separation process in which the vaporization temperature is less than 160° C.

2. The process according to claim 1, wherein the pressure in the separation apparatus is from 0.001 mbar to 10 mbar, preferably from 0.01 mbar to 1 mbar.

3. The process according to claim 1, wherein the average residence time of the mixture to be separated in the separation apparatus is from 30 to 1800 seconds, preferably from 50 to 1600 seconds.

4. A low-monomer polymethylenepolyphenylene polyisocyanate mixture comprising less than 0.4% by weight of 2-ring methylenediphenyl diisocyanate (MDI), comprising
20-45% by weight of 3-ring MDI,
10-30% by weight of 4-ring MDI and
30-60% by weight of ≥ 5-ring and higher MDI.

5. The low-monomer PMDI mixture according to claim 4 which has a viscosity of from 3 to 150 Pa s at 25°C and from 100 to 7000 mPa s at 50°C.

6. The low-monomer PMDI mixture according to claim 4 or 5 which has a functionality of from 3.8 to 4.4.

7. The use of a low-monomer PMDI mixture according to any of claims 4 to 6 for producing one-component polyurethane foam and/or two-component polyurethane foam.

8. An isocyanate component for producing one-component polyurethane foams and/or two-component polyurethane foams, which comprises
i) a low-monomer PMDI mixture according to any of claims 4 to 6 and
ii) diluents.

9. An isocyanate component for producing one-component polyurethane foams and/or two-component polyurethane foams, which comprises
i) a low-monomer PMDI mixture according to any of claims 4 to 6,
ii) isocyanate prepolymers obtainable by reacting a polyisocyanate with polyols and
iii) if appropriate, diluents.

10. An isocyanate component for producing one-component polyurethane foams, which comprises
i) a low-monomer PMDI mixture according to any of claims 4 to 6,
ii) polyisocyanate-silane prepolymers obtainable by reacting a polyisocyanate with polyols and subsquently reacting the product with silanes and
iii) if appropriate, diluents.

## Revendications

1. Procédé de préparation d'un mélange pauvre en monomères contenant des polyméthylène-polyphénylènepolyisocyanates (PMDI), dans lequel
a) on prépare un mélange PMDI comportant
35 ± 17% en poids de méthylènediphényldiisocyanate (MDI) à deux noyaux,
26 ± 6% en poids de MDI à trois noyaux,
12 ± 4% en poids de MDI à quatre noyaux,
5 ± 3% en poids de MDI à cinq noyaux,
23 ± 10% en poids de MDI à six noyaux et plus,
et ensuite
b) on soumet le mélange PMDI à un procédé de séparation par distillation, la température d'évaporation étant inférieure à 160°C.

2. Procédé suivant la revendication 1, **caractérisé en ce que** la pression dans le dispositif de séparation est de 0,001 mbar à 10 mbars, de préférence de 0,01 mbar à 1 mbar.

3. Procédé suivant la revendication 1, **caractérisé en ce que** le temps de séjour moyen du mélange à séparer dans le dispositif de séparation est de 30 à 1800 secondes, de préférence de 50 à 1600 secondes.

4. Mélange de polyméthylène-polyphénylènepolyisocyanate pauvre en monomères, contenant moins de 0,4% en poids de méthylènediphényldiisocyanate (MDI) à deux noyaux, et contenant
20 à 45% en poids de MDI à trois noyaux,
10 à 30% en poids de MDI à quatre noyaux, et
30 à 60% en poids de MDI à cinq noyaux et plus.

5. Mélange PMDI pauvre en monomères suivant la revendication 4, **caractérisé en ce qu'**il présente une viscosité à 25°C de 3 à 150 Pas et à 50°C de 100 à 7000 mPas.

6. Mélange PMDI pauvre en monomères suivant la revendication 4 ou 5, **caractérisé en ce qu'**il présente une fonctionnalité de 3,8 à 4,4.

7. Utilisation d'un mélange PMDI pauvre en monomères suivant l'une des revendications 4 à 6, pour la préparation d'une mousse de polyuréthanne à un composant et/ou d'une mousse de polyuréthanne à deux composants.

8. Composant isocyanate pour la préparation de mousses de polyuréthanne à un composant et/ou de mousses de polyuréthanne à deux composants, contenant
i) un mélange PMDI pauvre en monomères suivant l'une des revendications 4 à 6, et
ii) un diluant.

9. Composant isocyanate pour la préparation de mousses de polyuréthanne à un composant et/ou de mousses de polyuréthanne à deux composants, contenant
i) un mélange PMDI pauvre en monomères suivant l'une des revendications 4 à 6,
ii) des prépolymères d'isocyanate que l'on peut obtenir par réaction d'un polyisocyanate avec des polyols, et
iii) éventuellement un diluant.

10. Composant isocyanate pour la préparation de mousses de polyuréthanne à un composant et/ou de mousses de polyuréthanne à deux composants, contenant
i) un mélange PMDI pauvre en monomères suivant l'une des revendications 4 à 6,
ii) des prépolymères de polyisocyanatesilane, que l'on peut obtenir par réaction d'un polyisocyanate avec des polyols et ensuite réaction avec des silanes,
iii) éventuellement un diluant.
